# EUROPEAN PATENT APPLICATION

(11) **EP 3 858 384 A1**
(43) Date of publication of application: **04.08.2021**
(21) Application number: 19866728.9
(22) Date of filing: 29.09.2019
(51) Int. Cl.: A61K 39/395, C07K 16/30

(54) **COMBINATION THERAPY OF CLDN18 ANTIBODY AND CHEMOTHERAPY DRUGS**

(30) Priority: 30.09.2018 CN 201811159863; 19.10.2018 CN 201811224605
(71) Applicant: CAFA THERAPEUTICS LIMITED, Dublin D01 AE27 (IE)
(72) Inventor: LI, Zonghai, Shanghai 200231 (CN); WANG, Huamao, Shanghai 200231 (CN)
(74) Representative: Cabinet Laurent & Charras
(86) International application number: PCT/CN2019/109214
(87) International publication number: WO 2020/063988

(57) **Abstract**

A method for treating CLDN18-positive tumors, wherein an antibody that specifically recognizes CLDN18 and chemotherapy drugs are administered to a subject in need thereof. The chemotherapy drugs comprise: 5-fluorouracil, a prodrug thereof, or an active metabolite thereof, and oxaliplatin, a prodrug thereof, or an active metabolite thereof; or 5-fluorouracil, a prodrug thereof, or an active metabolite thereof, oxaliplatin, a prodrug thereof, or an active metabolite thereof, and a taxane drug. A kit combination for treating CLDN18 positive tumors. Compared with the existing technology, the treatment method and the kit combination have greatly enhanced tumor suppression rates and better anti-tumor effects. In addition, the present invention may reduce the use of chemotherapy drugs, and improve the safety of medication and improve tolerance of patients.

## Description

The present application claims the priority of a Chinese patent application, the application date of which is September 30, 2018, the application number of which is 201811159863.8, and the subject matter of which is "combination therapy of CLDN18.2 antibody and chemotherapeutic drug", and the priority of a Chinese patent application, the application date of which is October 19, 2018, the application number of which is 201811224605.3, the subject matter of which is "combination therapy of CLDN18 antibody and chemotherapeutic drug". The contents of said preceding applications are incorporated herein by reference in its entirety.

### Technical field

The present invention relatess to the field of biomedicine, and in particular, to a combination therapy of CLDN18 antibody and chemotherapeutic drugs.

### Background

Gastric cancer is one of the cancers with a high incidence worldwide. According to statistics from the Cancer Control Project of the WHO, there are up to 7 million patients die of cancer worldwide each year, of which 700,000 patients die of gastric cancer.

It was reported by Ganymed (German) that a combination of EOX (epirubicin, oxaliplatin and capecitabine) chemotherapeutic drugs and an antibody targeting Claudin 18.2 were used in combination to treat gastric cancer. In EP2852408B, it was also reported that a combination of EOF (epirubicin, 5-fluoropyrimidine and oxaliplatin) chemotherapeutic and an antibody targeting cell junction claudin (Claudin or CLDN) 18.2 were used in combination in mice to treat gastric cancer.

However, chemotherapeutic drugs usually have greater cytotoxicity and are poorly tolerated by patients, therefore the use of multiple chemotherapeutic drugs brings great risks to clinical application.

### Summary of the invention

The purpose of the present invention is to provide a combination therapy of CLDN18 antibody and chemotherapeutic drugs, which is used to effectively treat diseases related to the expression of CLD18A.

In the first aspect of the present invention, a method for treating a CLDN18-positive tumors is provided, comprising administering an antibody that specifically recognize CLDN18, 5-fluorouracil or a prodrug or active metabolite thereof, and oxaliplatin or a prodrug or active metabolite thereof to an individual in need thereof; or administering an antibody that specifically recognize CLDN18, 5-fluorouracil or a prodrug or active metabolite thereof, oxaliplatin or a prodrug or active metabolite thereof and taxanes to an individual in need thereof.

In a specific embodiment, an antibody that specifically recognize CLDN18, 5-fluorouracil or a prodrug thereof, and oxaliplatin or a prodrug thereof are administered to an individual in need thereof; or an antibody that specifically recognize CLDN18, 5-fluorouracil or a prodrug thereof, oxaliplatin or a prodrug thereof and taxanes are administered to an individual in need thereof.

In a specific embodiment, the prodrug of 5-fluorouracil is capecitabine.

In a specific embodiment, the CLDN18 is CLDN18.2.

In a specific embodiment, the antibody specifically recognizes CLDN 18.2, instead of CLDN 18.1.

In a specific embodiment, the antibody against CLDN18, 5-fluorouracil or a prodrug or active metabolite thereof, oxaliplatin or a prodrug or active metabolite thereof are administered without a particular order.

In a specific embodiment, 5-fluorouracil or a prodrug or active metabolite thereof, oxaliplatin, the antibody against CLDN18 are administered on the same day.

In a specific embodiment, the administration dosage of the antibody against CLDN18 is 500-1200 mg/m²/time. In a preferred example, the administration dosage is 500-1000 mg/m²/time. In a preferred example, the administration dosage is 500-900 mg/m²/time. In a preferred example, the administration dosage is 600-800 mg/m²/time.

In a specific embodiment, the administration dosage of the antibody against CLDN18 is 20-300 mg/kg/time. In a preferred example, the administration dosage is 20-100 mg/kg/time. In a preferred example, the administration dosage is 20-50 mg/kg/time.

In a specific embodiment, the administration dosage of 5-fluorouracil or a prodrug or active metabolite thereof is 300-900 mg/m²/time.

In a specific embodiment, 5-fluorouracil is administered at a dosage of 300-800 mg/m²/time.

In a specific embodiment, capecitabine is administered at 600-700 mg/m²/time. In a preferred example, capecitabine is administered at 625 mg/m²/time.

In a specific embodiment, the administration dosage of 5-fluorouracil or a prodrug or active metabolite thereof is 50-70 mg/kg/time. In a preferred example, 5-fluorouracil is administered at a dosage of 50-70 mg/kg/time. In a more preferred example, the administration dosage of 5-fluorouracil is 50-60 mg/kg/time.

In a specific embodiment, the administration dosage of oxaliplatin or a prodrug or active metabolite thereof is 50-300 mg/m²/time. In a preferred example, oxaliplatin is administered at 100-200 mg/m²/time. In a more preferred example, oxaliplatin is administered at 130 mg/m²/time.

In a specific embodiment, the administration dosage of oxaliplatin or a prodrug or active metabolite thereof is 1-10 mg/kg/time. In a preferred example, oxaliplatin is administered at 1 to 5 mg/kg/time.

In a specific embodiment, the antibody that specifically recognizes CLDN18 is a humanized antibody or a chimeric antibody.

In a specific embodiment, the antibody that specifically recognizes CLDN18 comprises:
HCDR1 shown in the amino acid sequence of SEQ ID NO: 16, HCDR2 shown in the amino acid sequence of SEQ ID NO: 17, 40, or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 22, HCDR2 shown in the amino acid sequence of SEQ ID NO: 23, HCDR3 shown in the amino acid sequence of SEQ ID NO: 24, and LCDR1 shown in the amino acid sequence of SEQ ID NO: 25, LCDR2 shown in the amino acid sequence of SEQ ID NO: 26, LCDR3 shown in the amino acid sequence of SEQ ID NO: 27; or
HCDR1 shown in the amino acid sequence of SEQ ID NO: 28, HCDR2 shown in the amino acid sequence of SEQ ID NO: 29, HCDR3 shown in the amino acid sequence of SEQ ID NO: 30, and LCDR1 shown in the amino acid sequence of SEQ ID NO: 31, LCDR2 shown in the amino acid sequence of SEQ ID NO: 32, LCDR3 shown in the amino acid sequence of SEQ ID NO: 33; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48; or
HCDR1 shown in the amino acid sequence of SEQ ID NO: 49, HCDR2 shown in the amino acid sequence of SEQ ID NO: 50, HCDR3 shown in the amino acid sequence of SEQ ID NO: 51, and LCDR1 shown in the amino acid sequence of SEQ ID NO: 52, LCDR2 shown in the amino acid sequence of SEQ ID NO: 53, LCDR3 shown in the amino acid sequence of SEQ ID NO: 54.

In a specific embodiment, the antibody that specifically recognizes CLDN18 comprises:
HCDR1 shown in the amino acid sequence of SEQ ID NO: 16, HCDR2 shown in the amino acid sequence of SEQ ID NO: 17 or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48.

In a specific embodiment, the antibody that specifically recognizes CLDN18 comprises:
the heavy chain variable region shown in the amino acid sequence of SEQ ID NO: 2, 9 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 4 and the light chain variable region shown in the amino acid sequence of SEQ ID NO: 3; or
the heavy chain variable region shown in the amino acid sequence of SEQ ID NO: 6 and the light chain variable region shown in the amino acid sequence of SEQ ID NO: 5; or
the heavy chain variable region shown in the amino acid sequence of SEQ ID NO: 55 and the light chain variable region shown in the amino acid sequence of SEQ ID NO: 56; or
the heavy chain variable region shown in the amino acid sequence of SEQ ID NO: 55 and the light chain variable region shown in the amino acid sequence of SEQ ID NO: 56; or
the heavy chain variable region shown in the amino acid sequence of SEQ ID NO: 57 and the light chain variable region shown in the amino acid sequence of SEQ ID NO: 58.

In a specific embodiment, the antibody that specifically recognizes CLDN18 comprises:
the heavy chain variable region shown in the amino acid sequence of SEQ ID NO: 2 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56.

In a preferred embodiment, the antibody that specifically recognizes CLDN18 comprises:
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56.

In a specific embodiment, the antibody that specifically recognizes CLDN18 is selected from:
(a) the heavy chain encoded by the nucleotide sequence shown in SEQ ID NO: 59 and the light chain encoded by the nucleotide sequence shown in SEQ ID NO: 60; or,
(b) the heavy chain amino acid sequence shown in SEQ ID NO: 64 and the light chain amino acid sequence shown in SEQ ID NO: 63.

In a specific embodiment, the CLDN18-positive tumor includes: gastric cancer, pancreatic cancer, esophageal cancer and lung cancer.

In a specific embodiment, the taxanes are selected from the group consisting of paclitaxel, albumin-bound paclitaxel and docetaxel; preferably, albumin-bound paclitaxel.

In the second aspect of the present invention, a combination of kits for treating a CLDN18-positive tumor is provided. The combination of kits includes kit A and kit B. The kit A includes an antibody that specifically recognizes CLDN18. The kit B includes chemotherapeutic drugs, and the chemotherapeutic drugs are: 5-fluorouracil or a prodrug or active metabolite thereof, oxaliplatin or a prodrug or active metabolite thereof; or 5-fluorouracil or a prodrug or active metabolite thereof, oxaliplatin or a prodrug or active metabolite thereof, and taxanes.

In a specific embodiment, the kit A and the kit B are administered to an individual in need thereof, wherein the kit B includes 5-fluorouracil or a prodrug thereof, and oxaliplatin or a prodrug thereof; or the kit A and the kit B are administered to an individual in need thereof, wherein the kit B includes 5-fluorouracil or a prodrug thereof, oxaliplatin or a prodrug thereof, and taxanes.

In a specific embodiment, the prodrug of 5-fluorouracil is capecitabine.

In a specific embodiment, the CLDN18 is CLDN18.2.

In a specific embodiment, the antibody specifically recognizes CLDN 18.2, instead of CLDN 18.1.

In a specific embodiment, the kit A and the kit B are administered without a particular order.

In a specific embodiment, the kit B comprising 5-fluorouracil or a prodrug or active metabolite thereof, oxaliplatin, and the kit A comprising an antibody to CLDN18 are administered on the same day.

In a specific embodiment, in the kit A, the administration dosage of the antibody to CLDN18 is 500-1200 mg/m²/time. In a preferred embodiment, the administration dosage is 500-1000 mg/m²/time. In a preferred embodiment, the administration dosage is 500-900 mg/m²/time. In a preferred embodiment, the administration dosage is 600-800 mg/m²/time.

In a specific embodiment, in the kit A, the administration dosage of the antibody to CLDN18 is 20-300 mg/kg/time. In a preferred embodiment, the administration dosage is 20-100 mg/kg/time. In a preferred embodiment, the administration dosage is 20-50 mg/kg/time.

In a specific embodiment, in the kit B, the administration dosage of 5-fluorouracil or a prodrug or active metabolite thereof is 300-900 mg/m²/time.

In a specific embodiment, 5-fluorouracil is administered at a dosage of 300-800 mg/m²/time.

In a specific embodiment, capecitabine is administered at 600-700 mg/m²/time. In a preferred embodiment, capecitabine is administered is 625 mg/m²/time.

In a specific embodiment, in the kit B, the administration dosage of 5-fluorouracil or a prodrug or active metabolite thereof is 50-70 mg/kg/time. In a preferred embodiment, 5-fluorouracil is administered at a dosage of 50-70 mg/kg/time. In a more preferred embodiment, the dosage of 5-fluorouracil is 50-60 mg/kg/time.

In a specific embodiment, in the kit B, the administration dosage of oxaliplatin or a prodrug or active metabolite thereof is 50-300 mg/m²/time. In a preferred embodiment, oxaliplatin is administered at 100-200 mg/m²/time. In a more preferred embodiment, oxaliplatin is administered 130 mg/m²/time.

In a specific embodiment, in the kit B, the administration dosage of oxaliplatin or a prodrug or active metabolite thereof is 1-10 mg/kg/time. In a preferred embodiment, oxaliplatin is administered at 1 to 5 mg/kg/time.

In a specific embodiment, in the kit A, the antibody that specifically recognizes CLDN18 is a humanized antibody or a chimeric antibody.

In a specific embodiment, in the kit A, the antibody that specifically recognizes CLDN18 comprises:
HCDR1 shown in the amino acid sequence of SEQ ID NO: 16, HCDR2 shown in the amino acid sequence of SEQ ID NO: 17, 40, or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 22, HCDR2 shown in the amino acid sequence of SEQ ID NO: 23, HCDR3 shown in the amino acid sequence of SEQ ID NO: 24, and LCDR1 shown in the amino acid sequence of SEQ ID NO: 25, LCDR2 shown in the amino acid sequence of SEQ ID NO: 26, LCDR3 shown in the amino acid sequence of SEQ ID NO: 27; or
HCDR1 shown in the amino acid sequence of SEQ ID NO: 28, HCDR2 shown in the amino acid sequence of SEQ ID NO: 29, HCDR3 shown in the amino acid sequence of SEQ ID NO: 30, and LCDR1 shown in the amino acid sequence of SEQ ID NO: 31, LCDR2 shown in the amino acid sequence of SEQ ID NO: 32, LCDR3 shown in the amino acid sequence of SEQ ID NO: 33; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48; or
HCDR1 shown in the amino acid sequence of SEQ ID NO: 49, HCDR2 shown in the amino acid sequence of SEQ ID NO: 50, HCDR3 shown in the amino acid sequence of SEQ ID NO: 51, and LCDR1 shown in the amino acid sequence of SEQ ID NO: 52, LCDR2 shown in the amino acid sequence of SEQ ID NO: 53, LCDR3 shown in the amino acid sequence of SEQ ID NO: 54.

In a specific embodiment, in the kit A, the antibody that specifically recognizes CLDN18 comprises:
HCDR1 shown in the amino acid sequence of SEQ ID NO: 16, HCDR2 shown in the amino acid sequence of SEQ ID NO: 17 or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39.

In a specific embodiment, in the kit A, the antibody that specifically recognizes CLDN18 comprises:
the heavy chain variable region shown in the amino acid sequence of SEQ ID NO: 2, 9 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 4 and the light chain variable region shown in the amino acid sequence of SEQ ID NO: 3; or
the heavy chain variable region shown in the amino acid sequence of SEQ ID NO: 6 and the light chain variable region shown in the amino acid sequence of SEQ ID NO: 5; or
the heavy chain variable region shown in the amino acid sequence of SEQ ID NO: 55 and the light chain variable region shown in the amino acid sequence of SEQ ID NO: 56; or
the heavy chain variable region shown in the amino acid sequence of SEQ ID NO: 57 and the light chain variable region shown in the amino acid sequence of SEQ ID NO: 58.

In a specific embodiment, in the kit A, the antibody that specifically recognizes CLDN18 comprises:
the heavy chain variable region shown in the amino acid sequence of SEQ ID NO: 2 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56.

In a preferred embodiment, in the kit A, the antibody that specifically recognizes CLDN18 comprises:
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 15 and the light chain variable region shown in the amino acid sequence of SEQ ID NO: 13;
the heavy chain variable region shown in the amino acid sequence of SEQ ID NO: 55 and the light chain variable region shown in the amino acid sequence of SEQ ID NO: 56.

In a specific embodiment, in the kit A, the antibody that specifically recognizes CLDN18 comprises
(a) the heavy chain encoded by the nucleotide sequence shown in SEQ ID NO: 59 and the light chain encoded by the nucleotide sequence shown in SEQ ID NO: 60; or
(b) the heavy chain amino acid sequence shown in SEQ ID NO: 64 and the light chain amino acid sequence shown in SEQ ID NO: 63.

In a specific embodiment, the CLDN18-positive tumor includes: gastric cancer, pancreatic cancer, esophageal cancer and lung cancer.

In a specific embodiment, in the kit B, the taxanes are selected from the group consisting of paclitaxel, albumin-bound paclitaxel and docetaxel; preferably, albumin-bound paclitaxel.

In the third aspect of the present invention, a pharmaceutical composition for treating a CLDN18-positive tumor is provided, wherein the pharmaceutical composition comprises an antibody that specifically recognizes CLDN18 and chemotherapeutic drugs, and the chemotherapeutic drugs are 5-fluorouracil or a prodrug or active metabolite thereof, oxaliplatin or a prodrug or active metabolite thereof; or, 5-fluorouracil or a prodrug or active metabolite thereof, oxaliplatin or a prodrug or active metabolite thereof, and taxanes, as described above; and the antibody that specifically recognizes CLDN18 is the antibody as described above.

In the fourth aspect of the present invention, a use of the above combination of kits or the above pharmaceutical composition in the preparation of a medicament for treating a CLDN18-positive tumor is provided.

In the fifth aspect of the present invention, the above combination of kits or pharmaceutical composition is provided for treating a CLDN18 positive tumor.

### Description of drawings

Figure 1 is a statistical chart of tumor volume of BALB/c nude mice bearing human gastric cancer PDX-GA0006 model after being treated with different treatments;
Figure 2 is a statistics chart of body weight of BALB/c nude mice bearing human gastric cancer PDX-GA0006 model after being treated with different treatments;
Figure 3 is a picture of tumors of BALB/c nude mice bearing human gastric cancer PDX-GA0006 model after being treated with different treatments;
Figure 4 is a statistical chart of tumor weight of BALB/c nude mice bearing human gastric cancer PDX-GA0006 model after being treated with different treatments. Note: Compared with the solvent group, * means P ≤ 0.05, ** means P ≤ 0.01, *** means P ≤ 0.001;
Figure 5 shows the anti-tumor effects of different dosages of antibodies;
Figure 6 shows the effects of different dosages of antibodies on the body weight of mice.

### Modes for carrying out the invention

After in-depth research and repeated experiments, the inventors obtained a method for treating a tumor with an antibody targeting CLDN 18.2, 5-fluoropyrimidine and oxaliplatin, which is a tumor expressing claudin 18A2, especially for treating gastric cancer, pancreatic cancer, esophageal cancer, lung cancer treatment.

The antibody of the present invention is an antibody that specifically binds to an epitope present on claudin 18A2 (CLDN18A2), and includes polyclonal antibodies and monoclonal antibodies, preferably monoclonal antibodies. Monoclonal antibodies contemplated in the present invention include IgA, IgG1-4, IgE, IgM and IgD antibodies. In one embodiment, the antibody is an IgG1 antibody. In addition, in some other alternatives, the antibody can also be an IgG3 antibody, such as IgG3K or IgG3λ isotype; and can also be an IgG4 antibody, such as IgG4K or IgG4λ isotype; or can be an IgA1 or IgA2 antibody, or an IgM antibody. In certain embodiments, the administration dosage of the antibody to CLD18A2 is 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300/kg/time; 1, 2, 3, 4, 5, 6 or 7 times per week; injected for 1 week, or continuously injected for 2, 3, 4, 5 or 6 weeks. In certain embodiments, the antibody to CLD18A2 is administered at a dosage of 500, 525, 550, 575, 600, 625, 650, 675, 700, 725, 750, 775, 800, 825, 850, 875, 900, 925, 950, 975, 1000, 1050, 1100, 1150, 1200 mg/m²/time; 1, 2, 3, 4, 5 or 6 times per week; injected for 1 week, or continuously injected for 2, 3, 4, 5 or 6 weeks.

For easily understanding the present invention, some terms are defined below.

The term "CLDN18.2" includes isotypes, mammalian (e.g., human) CLDN18.2, post-translational modified variants of human CLDN18.2, isoforms and interspecies homologues, and analogues comprising at least one common epitope with CLDN18.2. The amino acid sequence of CLDN18.2 (for example, human CLDN18.2) is known in the art, which is shown in the NCBI database. The terms "CLDN18A2", "CLDN18.2" and "Clindrin 18.2" can be used interchangeably herein, and the terms "CLDN18A1", "CLDN18.1" and "Cldudin 18.1" can be used interchangeably.

The term "oxaliplatin" as used herein refers to the compound [(1R,2R)-cyclohexane-1,2-diamine](ethanedioic acid (2-)-O,O')platinum. In some embodiments, oxaliplatin is injected 1, 2, 3, 4, 5, 6, 7, 8, 9, 10 mg/kg/time, 1, 2, 3, 4, 5, 6 or 7 injections per week, injected for 1 week, or continuously injected for 2, 3, 4, 5 or 6 weeks. In certain embodiments, oxaliplatin is injected 50, 60, 70, 80, 90, 100, 110, 120, 130, 140, 150, 160, 170, 180, 190, 200, 210, 220, 230, 240, 250, 260, 270, 280, 290, 300 mg/m²/time, 1, 2, 3, 4, 5, 6 or 7 times per week, injected for 1 week, or continuously injected for 2, 3 , 4, 5 or 6 weeks.

The term "5-fluoropyrimidine" as used herein refers to the compound 5-fluoro-1H-pyrimidine-2,4-dione. In certain embodiments, 5-fluorouracil or a prodrug or active metabolite thereof is administered in an amount of 50, 51, 52, 53, 54, 55, 56, 57, 58, 59, 60, 61, 62, 63, 64, 65, 66, 67, 68, 69, 70 mg/kg/time, 1, 2, 3, 4, 5, 6, or 7 times per week, injected for 1 week, or continuously injected for 2, 3, 4 , 5 or 6 weeks. In some embodiments, 5-fluorouracil or a prodrug or active metabolite thereof is administered in an amount of 300, 325, 350, 375, 400, 425, 450, 475, 500, 525, 550, 575, 600, 625, 650, 675, 700, 725, 750, 775, 800, 825, 850, 875, 900 mg/m²/time, 1, 2, 3, 4, 5, 6, or 7 times per week, injected for 1 week, or continuously injected for 2, 3, 4, 5 or 6 weeks. In certain embodiments, the prodrug of 5-fluorouracil is capecitabine. In certain embodiments, the capecitabine or a prodrug or active metabolite thereof is administered in an amount of 600, 605, 610, 615, 620, 625, 630, 635, 640, 645, 650, 655, 660 , 665, 670, 675, 680, 685, 690, 695, 700 mg/m²/time, 1, 2, 3, 4, 5, 6, or 7 times per week, injected for 1 week, or continuously injected for 2, 3, 4, 5 or 6 weeks.

The term "epirubicin" as used herein refers to the compound 10-[(3-amino-2,3,6-tideoxy-A-L-alpha-hexapyranosyl)oxy]-7,8,9,10-tetrahydro-6,8,11-trihydroxy-8-(hydroxyacetyl)-methoxy-(8S-CIS)-tetracene-5,12-dione.

The term "antibody" refers to a glycoprotein comprising at least two heavy (H) chains and two light (L) chains or antigen-binding fragments thereof interconnected by disulfide bonds. The term "antibody" also includes all recombinant forms of antibodies (especially the antibodies described herein), such as antibodies expressed in prokaryotic cells, unglycosylated antibodies, and antigen-bound fragments of an antibody and derivatives described below. Each heavy chain consists of a heavy chain variable region (abbreviated herein as VH) and a heavy chain constant region. Each light chain consists of a light chain variable region (abbreviated herein as VL) and a light chain constant region. VH and VL can be further subdivided into hypervariable regions called complementarity determining regions (CDR), which are interspersed in more conserved regions called framework regions (FR). Each VH and VL consists of three CDRs and four FRs, arranged in the following order from the amino terminus to the carboxy terminus: FR1, CDR1, FR2, CDR2, FR3, CDR3, FR4. The variable regions of the heavy and light chains contain binding domains that interact with antigens. The constant region of an antibody can mediate the binding of the immunoglobulin to host tissues or factors, which include various cells of the immune system (such as effector cells) and the first component of the classical complement system (C1q).

The term "monoclonal antibody" as used herein refers to a preparation of antibody molecules consisting of single molecules. A monoclonal antibody exhibits a single binding specificity and affinity for a specific epitope. In one embodiment, monoclonal antibodies are produced by hybridomas, which include B cells derived from non-human animals (e.g., mice) fused with biochemical cells.

The antibody described herein can be a recombinant antibody, and the "recombinant antibody" includes all antibodies prepared, expressed, produced or isolated by recombinant means, such as (a) an antibody isolated from animals (such as mice) whose immunoglobulin genes are transgenic or transchromosome, or hybridomas prepared therefrom, (b) an antibody isolated from a host cell (such as a transfectionoma) transformed to express the antibody, (c) an antibody isolated from a recombinant combinatorial antibody library, and (d) an antibody prepared, expressed, produced or isolated by any other means involving the splicing of immunoglobulin gene sequences into DNA sequences.

The term "humanized antibody" herein refers to an antibody in which CDR sequences derived from the germline of another mammalian species (e.g., mouse) are grafted onto human framework sequences. Additional framework region modifications can also be made in human framework sequences and CDR sequences derived from the germline of another mammalian species.

The term "chimeric antibody" herein refers to an antibody in which the variable region sequence is derived from one species and the constant region sequence is derived from another species, for example, an antibody in which the variable region sequence is derived from a mouse antibody and the constant region sequence is derived from a human antibody.

The term "Fab" or "Fab region" herein includes polypeptides comprising VH, CHI, VL and CL immunoglobulin domains. Fab can refer to an isolated region, or a region in the context of a full-length antibody or antibody fragment. The term "Fc" or "Fc region" herein includes a polypeptide comprising the constant region of an antibody other than the immunoglobulin domain of the first constant region. Therefore, Fc refers to the last two constant region immunoglobulin domains of IgA, IgD, and IgG, the last three constant region immunoglobulin domains of IgE and IgM, and the flexible hinge at the N-terminus of these domains. For IgA and IgM, Fc may comprise the J chain. For IgG, Fc includes immunoglobulin domains Cγ2 and Cγ3 as well as a hinge between Cγ1 and Cγ2. Although the boundaries of the Fc region can be changed, the Fc region of a human IgG heavy chain is generally defined as comprising residues C226 or P230 at its carboxy terminus, where the numbering is based on the EU index of Kabat. For human IgG1, Fc is defined herein as comprising residue P232 to its carboxyl-terminus, where the numbering is according to the EU index in Kabat. Fc can refer to an isolated region, or an region in the context of an Fc polypeptide, such as an antibody.

The term "hinge" or "hinge region" or "hinge region of an antibody" herein includes a flexible polypeptide comprising amino acids between the first and second constant domains of an antibody. Structurally, in the IgGCH1 domain, the hinge ends at EU220, and in the IgGCH2 domain starts at EU237. Therefore, for IgG, the hinge of an antibody is defined herein as including positions 221 (D221 of IgG1) to 231 (A231 of IgG1), where the numbering is based on the EU index of Kabat.

The term "variant" as used herein includes antibody sequences that differ from the parent antibody sequence due to at least one amino acid modification compared with the parent antibody. The sequence of a variant antibody herein preferably has at least about 80%, preferably at least about 90%, and more preferably at least about 95% amino acid sequence identity with the parent antibody sequence. An antibody variant can refer to the antibody itself, a composition comprising the parent antibody, or the amino acid sequence encoding it.

The term "amino acid modification" herein includes amino acid substitutions, insertions and/or deletions in the polypeptide sequence. "Amino acid substitution" or "substitution" herein means to replace an amino acid at a specific position in the parent polypeptide sequence with another amino acid. For example, the substitution of R94K refers to the replacement of arginine at position 94 by lysine, which is a variant of the heavy chain variable framework region. For the previous example, 94K means that position 94 is replaced with lysine. For the purposes of the present application, multiple substitutions are usually separated by a slash. For example, R94K/L78V refers to a double variant that includes the substitutions R94K and L78V. "Amino acid insertion" or "insertion" as used herein means the addition of an amino acid at a specific position in the parent polypeptide sequence. For example, the insertion -94 indicates an insertion at position 94. "Amino acid deletion" or "deletion" as used herein means the removal of an amino acid at a specific position in the parent polypeptide sequence. For example, R94- means that the arginine at position 94 is deleted.

All positions of an immunoglobulin heavy chain constant region discussed in the present invention are numbered according to EU index of Kabat (Kabat et al., 1991, Sequences of proteins of immunological interest, 5th edition, United States Public Health Service, National Institutes of Health , Bethesda, integrated by reference). " EU index of Kabat" refers to the residue numbering of the human IgG1 EU antibody, as described in Edelman et al., 1969, Biochemistry 63:78-85.

The term "full-length antibody" as used herein includes the structures that constitute the natural biological form of an antibody, including variable and constant regions. For example, in most mammals, including humans and mice, full-length IgG antibodies are tetramers, consisting of two identical pairs of immunoglobulin chains, each of which has a light chain and a heavy chain. Each light chain contains immunoglobulin domains VL and CL, and each heavy chain contains immunoglobulin domains VH, CH1 (Cγ1), CH2 (Cγ2) and CH3 (Cγ3). In some mammals, such as camels and llamas, IgG antibodies can consist of only two heavy chains, each of which contains a variable domain connected to the Fc region.

Antibody fragments include but are not limited to: (i) Fab fragment consisting of VL, VH, CL and CH1 domains, including Fab' and Fab'-SH, (ii) Fd fragment consisting of VH and CH1 domains, (iii) Fv fragment consisting of VL and VH domains of a single antibody; (iv) dAb fragment consisting of a single variable region; (v) F(ab')2 fragment, a bivalent fragment containing two linked Fab fragments; (vi) single-chain Fv molecule antigen binding site; (vii) bispecific single-chain Fv dimer; (viii) "Dibody" or "tribody", a multivalent or multispecific fragment constructed by gene fusion; and (ix) scFv genetically fused with the same or different antibodies.

According to the determination of constant region gene, antibodies are classified as isotypes. Human constant light chains are divided into K (CK) and λ (Cλ) light chains. Heavy chains are classified into µ, δ, γ, α, or ε, and define isotypes of an antibody, IgM, IgD, IgG, IgA, and IgE, respectively. The IgG class is commonly used for therapeutic purposes. In humans, this class includes subclasses IgG1, IgG2, IgG3, and IgG4. In mice, this class includes subclasses IgG1, IgG2a, IgG2b, and IgG3. IgM has subclasses, including but not limited to IgM1 and IgM2. IgA has several subclasses, including but not limited to IgA1 and IgA2. Therefore, "isotype" as used herein means any class or subclass of an immunoglobulin defined by the chemical and antigenic characteristics of the constant region. The known isotypes of human immunoglobulins are IgG1, IgG2, IgG3, IgG4, IgA1, IgA2, IgM1, IgM2, IgD and IgE.

The term "ADCC" or "antibody-dependent cell-mediated cytotoxicity" as used herein includes a cell-mediated response in which non-specific cytotoxic cells expressing FcγR recognize the bound antibody on a target cell, thereby leading to the lysis of the target cell . In various aspects, enhanced ADCC effector function can refer to the enhanced potency or enhanced efficacy. The "titer" used in the experiment context refers to the concentration of the antibody (half-maximum effective concentration) when the EC50 of a specific therapeutic effect is observed. "Efficacy" used in the experiment context refers to the probable effector function of the antibody at the saturation level.

The term "ADCP" or "antibody-dependent cell-mediated phagocytosis" as used herein includes a cell-mediated response in which non-specific cytotoxic cells expressing FcγR recognize antibodies bound on a target cell, thereby leading to the phagocytosis of the target cell.

The term "CDC" or "complement-dependent cytotoxicity" as used herein includes a reaction in which one or more complement protein components recognize the antibody bound on a target cell, thereby leading to the lysis of the target cell.

The term "effector function" as used herein includes biochemical events caused by the interaction of the Fc region of an antibody with an Fc receptor or ligand. Effector functions include FcγR-mediated effector functions, such as ADCC and ADCP, and complement-mediated effector functions, such as CDC.

In present specification, the body surface area formula can be selected by Stevenson formula: body surface area (m²) = 0.0061 × height (cm) + 0.0128 × weight (kg) - 0.1529. For example: a person with a height of 168 cm and a weight of 55 kg, his body surface area can be calculated as: 0.0061 × 168 + 0.0128 × 55-0.1529 = 1.576 m². The formulas for calculating the body surface area of men and women are: Sₘₐₗₑ = 0.0057 × height + 0.0121 × weight + 0.0882, S_{female} = 0.0073 × height + 0.0127 × weight - 0.2106.

### Antibody against Claudin 18A2

Each of the heavy chain and light chain variable region sequences of an antibody listed in the present invention can bind to human claudin 18A2, and the heavy chain and light chain variable region sequences can be "mixed and matched" to produce a binding molecule against human claudin 18A2 of the present invention.

Variants of an antibody or fragments thereof that bind to human claudin 18A2 are also provided in the present invention. Therefore, the present invention also includes an antibody or fragments thereof, and the heavy chain or light chain variable region sequence of the antibody has at least 80% amino acid sequence identity with the heavy chain and/or light chain variable region sequence of the antibody disclosed in the present invention. Preferably, the amino acid sequence identity is at least 85%, more preferably at least 90%, preferably at least 95%, particularly 96%, more particularly 97%, even more particularly 98%, particularly 99%, including for example 80%, 81%, 82%, 83%, 84%, 85%, 86%, 87%, 88%, 89%, 90%, 91%, 92%, 93%, 94%, 95%, 96%, 97% , 98%, 99% and 100%. The identity of an amino acid sequence herein refers to the percentage of amino acid residues in the sequence that are identical to the humanized antibody or fragment thereof that binds to human claudin 18A2. Therefore, the sequence identity can be determined by standard methods commonly used to compare the similarity of amino acid positions of two polypeptides. Using computer programs such as BLAST or FASTA, the two polypeptides are aligned for the best match of the amino acids (along the full length of one or two sequences or along predetermined portions of one or both sequences). The program provides default open penalties and default gap penalties. A scoring matrix such as PAM250 can be used in combination with a computer program. For example, percent identity can be calculated as: the total number of identical matches multiplied by 100, then divided by the total length of the longer sequence in the matching span and the number of gaps introduced into the longer sequence in order to align the two sequences.

In certain embodiments, framework sequences can be used to engineer variable regions to produce variant antibodies. The variant antibodies of the present invention include variants in which the residues of the framework region in VH and/or VK are modified, for example, to improve the characteristics of the antibody. Generally, such modifications of framework region are conducted to reduce the immunogenicity of the antibody. For example, one method is to "back mutate" one or more framework region residues to the corresponding murine sequence, or "back mutate" one or more framework region residues to the corresponding germline sequence.

Therefore, in another aspect, the present invention provides a humanized antibody or fragments thereof that binds to human claudin 18A2, wherein the framework region of at least one heavy chain variable region of the humanized antibody or fragments thereof includes at least one amino acid modification in the corresponding framework region of the heavy chain variable region. Preferably, the amino acid modification is an amino acid substitution. Generally, no more than 5, preferably no more than 4, more preferably no more than 3, even more preferably no more than 2, and preferably no more than 1 amino acid modification are performed in the framework region.

The present invention also provides a humanized antibody and fragments thereof that bind to human claudin 18A2, which also comprises human heavy chain and/or light chain constant domains. The human heavy chain constant region can be selected from the group consisting of human immunoglobulins, IgG1, IgG2, IgG3, IgG4, IgA1, IgA2, IgM1, IgM2, IgD and IgE, and for human heavy chain constant region IgG, IgG1 is especially preferred. The human light chain constant region can be selected from the group consisting of human immunoglobulins K or λ constant regions, and the human K constant region is preferred. In some preferred embodiments, the humanized antibody or fragments thereof includes a human IgG1 heavy chain constant domain and a human light chain K constant domain. The present invention also provides a humanized antibody or fragments thereof that binds to human claudin 18A2, which comprises a human heavy chain and/or light chain constant region, wherein the human heavy chain constant region comprises CH1 of human IgG1, the hinge region of human IgG1, and an isotype variant of Fc region of human IgG3. Preferred humanized antibodies comprising isotype variants are full-length antibodies. Humanized antibodies or fragments thereof that comprise human IgG1 comprise a sequence comprising the heavy chain variable region sequence shown in SEQ ID NO: 12, 14, 15, 55 or 57, and the heavy chain variable region sequence shown in SEQ ID NO: 11, 13, 56 or 58, preferably, the combination of the heavy chain variable region shown in SEQ ID NO: 12 and the light chain variable region shown in SEQ ID NO: 11; or the heavy chain variable region sequence shown in SEQ ID NO: 14 or 15 and the light chain variable region sequence shown in SEQ ID NO: 13; or the combination of the the heavy chain variable region sequence shown in SEQ ID NO: 55 and the light chain variable region sequence shown in SEQ ID NO: 56.

The effector function is usually complement-dependent cytotoxicity (CDC) and/or C1q binding and/or antibody-dependent cell-mediated cytotoxicity (ADCC) and/or the binding affinity of antibodies to Fcγ receptors, preferably complement-dependent cells Toxicity (CDC) and/or antibody-dependent cell-mediated cytotoxicity (ADCC). CDC, Clq binding, ADCC and the binding affinity of an antibody to Fcγ receptors are measured by standard *in vitro* assays, which are known in the art and commercially available. Generally, ADCC is measured by a lactate dehydrogenase (LDH) release assay, and CDC is measured by an assay administered to cells.

Standard assays to assess the binding ability of antibodies, such as antibodies against human claudin 18A2 are known in the art and include, for example, ELISA, Western blot, and flow cytometry analysis. Suitable assays are described in detail in the examples. To assess binding, 293T cells stably transfected with CLD18A2, AGS cells stably transfected with CLD18A2, NCI-N87 cells stably transfected with CLD18A2, and BGC cells stably transfected with CLD18A2 can be used, and EC50 can be determined by using flow cytometry analysis.

Hereinafter, the preferred embodiments of the present invention will be described in detail with reference to the accompanying drawings. The examples are given to better describe the contents of the present invention, but the contents of the present invention are not limited to the examples. A skilled person in the art can make non-essential improvements and adjustments to the embodiments based on the above contents of the invention, which shall fall within the protection scope of the present invention.

### Example 1. Preparation of antibodies

The antibody targeting CLD18A2 selected in this example is a humanized antibody, which has a heavy chain variable region shown in SEQ ID NO: 15 and a light chain variable region shown in SEQ ID NO: 13. The nucleotide sequence of the light chain is shown in SEQ ID NO: 60, and the nucleotide sequence of the heavy chain is shown in SEQ ID NO: 59.

The amplification of the variable regions of the light chain and the heavy chain was performed according to the "step-out PCR" method described by Matz et al. (Nucleic Acids Research, 1999, Vol. 27, No. 6). The nucleotide sequence of the light chain (shown in SEQ ID NO: 60) and the nucleotide sequence of the heavy chain (shown in SEQ ID NO: 59) were cloned into eukaryotic expression vector by standard methods known to a skilled person in the art. 293fectin™ Transfection reagent (Invitrogen, 12347-019) or polyethyleneimine (PEI) (Sigma-Aldrich, 408727) was used to transiently transfect 293F cells at logarithmic growth phase. After 5-7 days of transfection, the culture supernatant was collected and subjected to affinity purification by Protein A to obtain the antibody against CLD18A2. Quantitative and qualitative analysis of the obtained antibody were conducted by SDS PAGE. The above eukaryotic expression was the vector pH or the vector pK used in CN101602808B.

### Example 2. In vivo efficacy experiment of subcutaneous transplanted tumor in mouse PDX model

A 2×2×2 mm gastric cancer PDX tumor mass was inoculated into BALB/c mice (the day of inoculation was D0). On D27 of subcutaneous inoculation, the tumor volume was 128 mm³ on average, thereby obtaining the human gastric cancer PDX-GA0006 model, or abbreviated as PDX model. The tumor-forming mice were randomly divided into 6 groups, namely solvent group, antibody group (Ab group), EOF group, EOF + Ab group, OF group, OF + Ab group. During the experiment, the general clinical symptoms of the animals were observed, and the body weight and tumor diameter were measured twice a week. On D51, the mice were euthanized. The manners of administration are listed as follows:
Solvent group: intraperitoneal injection, 3 times a week.
Ab group: antibody against CLD18A2 prepared in Example 1, 40 mg/kg, intraperitoneal injection, 3 times a week.
EOF group: a combination of epirubicin hydrochloride for intraperitoneal injection (1.25 mg/kg) + oxaliplatin for injection (3.25 mg/kg) + fluorouracil injection (56.25 mg/kg), once a week; solvent, intraperitoneal injection, 3 times a week.
EOF + Ab group: EOF (epirubicin hydrochloride (1.25 mg/kg) + oxaliplatin for injection (3.25 mg/kg) + fluorouracil injection (56.25 mg/kg)), once a week, the antibody against CLD18A2 (40 mg/kg), 3 times a week.
OF group: a combination of oxaliplatin for injection (3.25 mg/kg) + fluorouracil injection (56.25 mg/kg), once a week; solvent, intraperitoneal injection, 3 times a week.
OF + Ab group: OF (Oxaliplatin for injection (3.25 mg/kg) + fluorouracil injection (56.25 mg/kg)), once a week, and the antibody against CLD18A2 antibody (40 mg/kg), 3 times a week.

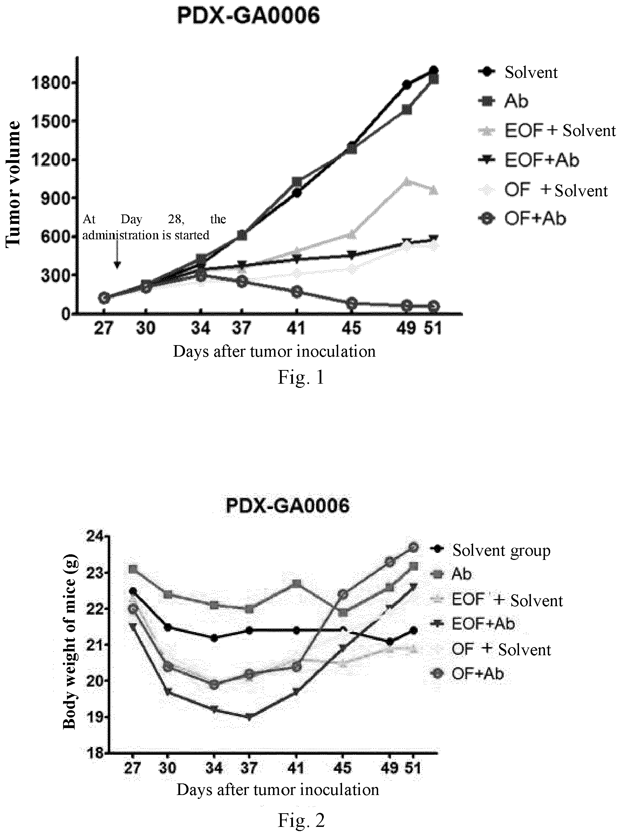

The results of the increase in tumor volume are shown in Figure 1. Compared with the EOF + Ab group, anti-tumor effects in the OF+Ab group were greatly enhanced when epirubicin was not used. The body weight of the mice is shown in Figure 2. The body weight of the OF+Ab group was slightly better than that of the EOF+Ab group.

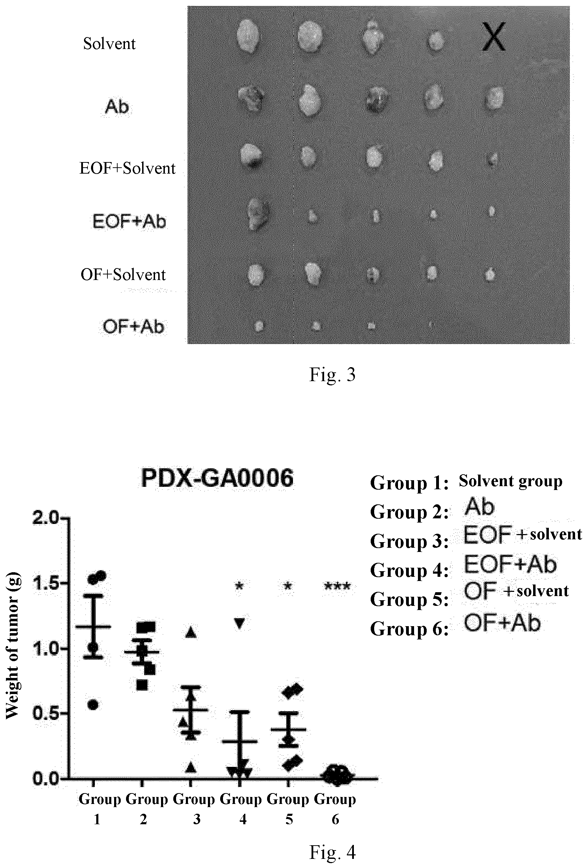

After the mice were euthanized, tumors were obtained and weighed. The results are shown in Figures 3 and 4. The tumor burden of mice in the solvent group was too large, and one mouse died at D51 after tumor inoculation. Compared with the solvent group, tumor inhibition rates of each group were: Ab group, 1.21%; EOF group, 48.70%; EOF + Ab group, 69.46%; OF group, 71.64%; OF + Ab group, 96.86%, and in this group, 1 out of 5 mice showed tumor regression (* p < 0.05; ** p < 0.01 or *** p < 0.001, One wayANOVA).

### Example 3. Analysis of the administration amount of antibody

According to Example 2, different dosage of antibodies were administered to tumor-forming mice. The administration manners are listed as follows:
1. AB1, administered with the antibody against CLD18A2 prepared in Example 1, at a dosage of 40 mg/kg, intraperitoneal injection, 3 times a week for 2 weeks.
2. AB2, administered with the antibody against CLD18A2 prepared in Example 1, at a dosage of 70 mg/kg, intraperitoneal injection, 3 times a week for 2 weeks.
3. AB3, administered with the antibody against CLD18A2 prepared in Example 1, at a dosage of 100 mg/kg, intraperitoneal injection, 3 times a week for 2 weeks.

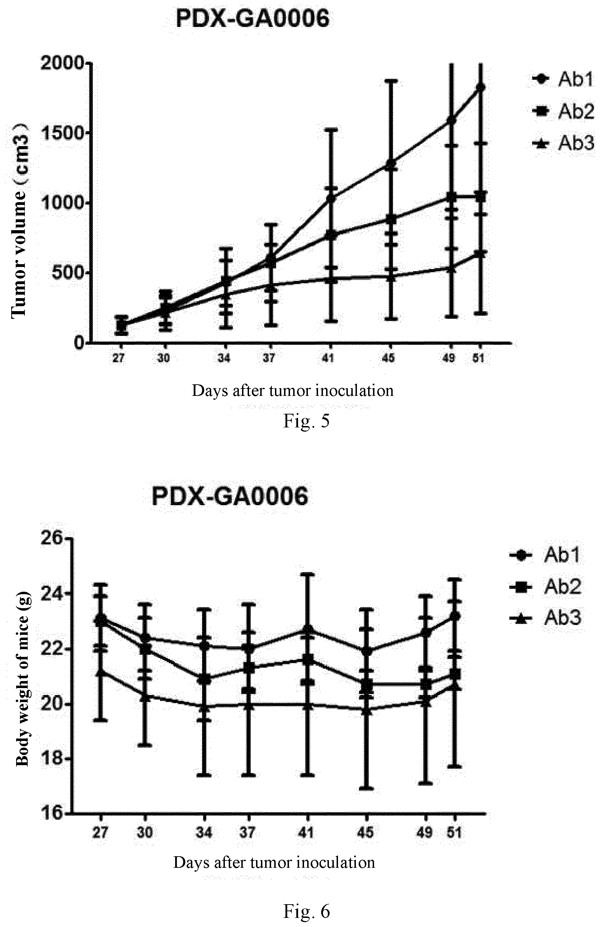

The results of the increase in tumor volume are shown in Figure 5, which shows that the administration in AB3 group exhibited better anti-tumor effects. The changes in the body weight of mice is shown in Figure 6. There is no significant change in body weight, indicating that the administration of large dosages of the antibody against CLD18A2 exhibited better anti-tumor effects without significant side effects.

In the above Examples, an humanized antibody against CLD18A2 was exemplarily used. A skilled person can use other antibodies based on the teachings of the present application, such as an antibody comprising the heavy chain variable region shown in SEQ ID NO: 12 and the light chain variable region shown in SEQ ID NO: 11, or an antibody comprising the heavy chain variable region shown in SEQ ID NO: 15 and the light chain variable region shown in SEQ ID NO: 13, or an antibody comprising the heavy chain variable region shown in SEQ ID NO: 55 and the light chain variable region shown in SEQ ID NO: 56, or an antibody comprising the heavy chain variable region shown in SEQ ID NO: 57 and the light chain variable region shown in SEQ ID NO: 58.

The sequences involved in the present invention are shown in the following table:

| SEQ ID NO: | Sequence |
|---|---|
| 1 | Divmtqspssltvtagekvtmscksssqsllnsgnqknyltwyqqkpgqppklliywastresgvpdrftgsgsgtdftltissv qaedlavyycqndysypltfgagtklelkr |
| 2 | Qvqlqqsgaelarpgasvkmsckasgytftsytmhwvkqrpgqglewigyinpssgytnynqkfkdkatltadkssstay mqlssltsedsavyycariyygnsfaywgqgttvtvss |
| 3 | QIVLTQSPAIMSASPGEKVTMTCSASSSISYMHWYQQKPGTSPKRWIYDTSKLAS GVPARFSGSGSGTSYSLTISSMEAEDAATYYCHQRSSYPYTFGGGTKLEIKR |
| 4 | QVQLQQSGPELVKPGALVKISCKASGYTFTSYDINWVKQRPGQGLEWIGWIYPGDG STKYNEKFKGKATLTADKSSSTAYMQLSSLTSENSAVYFCARGGYRYDEAMDYWG QGTTVTVSS |
| 5 | Divmtqspsslsvsagekvtmscksssqsllnsgnqknylawyqqkpgqppklliygastresgvpdrftgsgsgtdftltissvq aedlavyycqndhsypltfgagtklelkr |
| 6 | Qiqlvqsgpelkkpgetvkisckasgytftnygmnwvkqapgkglkwmgwintntgeptyaeefkgrfafsletsastaylqi nnlknedtatyfcarfsygnsfaywgqgttvtvss |
| 7 | Divmtqspssltvtpgekvtmtckssqslfnsgnqknyltwyqqrpgqppkmliywastresgvpdrftgsgsgtdftltissvq aedlavfycqnaysfpytfgggtkleikr |
| 8 | Dvqlqesgpdlvkpsqslsltctvtgysitsgynwhwirqfpgnkmewmgyihytgstnynpslrsrisitrdtsknqfflqlnsv ttddtatyyctriyngnsfpywgqgtsvtvss |
| 9 | Qvqlqqsgaelarpgasvkmsckasgytftsytmhwvkqrpgqglewigyidpssgytnynqkfkdkatltadkssstaymq lssltsedsavyycariyygnsfaywgqgttvtvss |
| 10 | Qvqlqqsgaelarpgasvkmsckasgytftsytmhwvkqrpgqglewigyinpasgytnynqkfkdkatltadkssstaymq lssltsedsavyycariyygnsfaywgqgttvtvss |
| 11 | Divmtqspdslavslgeratinckssqsllnsgnqknyltwyqqkpgqppklliywastresgvpdrfsgsgsgtdftltisslqae dvavyycqndysypltfgggtkveikr |
| 12 | Qvqlvqsgaevkkpgasvkvsckasgytftsytmhwvrqapgqglewmgyinpasgytnynqkfkdrvtmtrdtststaym elsslrsedtavyycariyygnsfaywgqgtlvtvss |
| 13 | Divmtqspdslavslgeratinckssqslfnsgnqknyltwyqqkpgqppklliywastresgvpdrfsgsgsgtdftltisslqae dvavyycqnaysfpytfgggtkleikr |
| 14 | Qvqlqesgpglvkpsqtlsltctvsggsissgynwhwirqppgkglewigyihytgstnynpalrsrvtisvdtsknqfslklssv taadtavyycariyngnsfpywgqgttvtvss |
| 15 | QVQLQESGPGLIKPSQTLSLTCTVSGGSISSGYNWHWIRQPPGKGLEWIGYIHYTGST NYNPALRSRVTISVDTSKNQFSLKLSSVTAADTAIYYCARIYNGNSFPYWGQGTTVT VSS |
| 16 | SYTMH |
| 17 | YINPSSGYTNYNQKFKD |
| 18 | IYYGNSFAY |
| 19 | KSSQSLLNSGNQKNYLT |
| 20 | WASTRES |
| 21 | QNDYSYPLT |
| 22 | SYDIN |
| 23 | WIYPGDGSTKYNEKFKG |
| 24 | GGYRYDEAMDY |
| 25 | SASSSISYMH |
| 26 | DTSKLAS |
| 27 | HQRSSYPYT |
| 28 | NYGMN |
| 29 | WINTNTGEPTYAEEFKG |
| 30 | FSYGNSFAY |
| 31 | KSSQSLLNSGNQKNYLA |
| 32 | GASTRES |
| 33 | QNDHSYPLT |
| 34 | SGYNWH |
| 35 | YIHYTGSTNYNPSLRS |
| 36 | IYNGNSFPY |
| 37 | KSSQSLFNSGNQKNYLT |
| 38 | WASTRES |
| 39 | QNAYSFPYT |
| 40 | YIDPSSGYTNYNQKFKD |
| 41 | YINPASGYTNYNQKFKD |
| 42 | yihytgstnynpalrs |
| 43 | SYWIN |
| 44 | NIYPSDSYTNYNQKFKD |
| 45 | SWRGNSFDY |
| 46 | KSSQSLLNSGNQKNYLT |
| 47 | WASTRES |
| 48 | QNDYSYPFT |
| 49 | NYGMN |
| 50 | WINTNTGEPTYAEEFKG |
| 51 | LGFGNAMDY |
| 52 | KSSQSLLNSGNQKNYLT |
| 53 | WASTRES |
| 54 | QNDYSYPLT |
| 55 | Qvqlqqpgaelvrpgasvklsckasgytftsywinwvkqrpgqglewigniypsdsytnynqkfkdkatltvdkssstaymql ssptsedsavyyctrswrgnsfdywgqgttltvss |
| 56 | Divmtqspssltvtagekvtmsckssqsllnsgnqknyltwyqqkpgqppklliywastresgvpdrftgsgsgtdftltissvqae dlavyycqndysypftfgsgtkleikr |
| 57 | Divmtqspssltvtagekvtmsckssqsllnsgnqknyltwyqqkpgqppklliywastresgvpdrftgsgsgtdftltissvqae dlavyycqndysypltfgagtklelkr |
| 58 | Qiqlvqsgpelkkpgetvkisckasgytftnygmnwvkqapgkglkwmgwintntgeptyaeefkgrfafsletsastaylqin nlknedtatyfcarlgfgnamdywgqgtsvtvss |
| 59 | Caggtgcagctgcaggagagcggccccggcctgatcaagcccagccagaccctgagcctgacctgcaccgtgagcggcggc agcAtcagcagcggctacaactggcactggatccggcagcccccggcaagggcctggagtggatcggctacatccactacac cggcagCaccaactacaaccccgccctgcggagccgggtgaccatcagcgtggacaccagcaagaaccagttcagcctgaag ctgagcagcgTgaccgccgccgacaccgccatctactactgcgcccggatctacaacggcaacagcttcccctactggggccag ggcaccaccgtgacCgtgagcagcgctagcaccaaaggcccatcggtcttccccctggcaccctcctccaagagcacctctggg ggcacagcggccctgggCtgcctggtcaaggactacttccccgaaccggtgacggtgtcgtggaactcaggcgccctgaccag cggcgtgcacaccttcccggctgTcctacagtcctcaggactctactccctcagcagcgtggtgaccgtgccctccagcagcttgg gcacccagacctacatctgcaacgtgaaTcacaagcccagcaacaccaaggtggacaagaaagttgagcccaaatcttgtgaca aaactcacacatgcccaccgtgcccagcacctgAactcctggggggaccgtcagtcttcctcttccccccaaaacccaaggacac cctcatgatctcccggacccctgaggtcacatgcgtggtGgtggacgtgagccacgaagaccctgaggtcaagttcaactggtac gtggacggcgtggaggtgcataatgccaagacaaagccgcggGaggagcagtacaacagcacgtaccgtgtggtcagcgtcct caccgtcctgcaccaggactggctgaatggcaaggagtacaagtgcaagGtctccaacaaagccctcccagcccccatcgagaa aaccatctccaaagccaaagggcagccccgagaaccacaggtgtacaccctgccCccatcccgggatgagctgaccaagaacc aggtcagcctgacctgcctggtcaaaggcttctatcccagcgacatcgccgtggagtgggaGagcaatgggcagccggagaaca actacaagaccacgcctcccgtgctggactccgacggctccttcttcctctatagcaagctcaccgtgGacaagagcaggtggcag caggggaacgtcttctcatgctccgtgatgcatgaggctctgcacaaccactacacgcagaagagcctctccctgtctccgggtaaa |
| 60 | Gacatcgtgatgacccagagccccgacagcctggccgtgagcctgggcgagcgggccaccatcaactgcaagagcagccaga gcctgtTcaacagcggcaaccagaagaactacctgacctggtaccagcagaagcccggccagccccccaagctgctgatctact gggccagcaccCgggagagcggcgtgcccgaccggttcagcggcagcggcagcggcaccgacttcaccctgaccatcagca gcctgcaggccgaggacGtggccgtgtactactgccagaacgcctacagcttccccctacaccttcggcggcggcaccaagctgg agatcaagcggacggtggctgcacCatctgtcttcatcttcccgccatctgatgagcagttgaaatctggaactgcctctgttgtgtgc ctgctgaataacttctatcccagagaggccaaAgtacagtggaaggtggataacgccctccaatcgggtaactcccaggagagtg tcacagagcaggacagcaaggacagcacctacagcctCagcagcaccctgacgctgagcaaagcagactacgagaaacacaa agtctacgcctgcgaagtcacccatcagggcctgagctcgcccgtcacaaagagcttcaacaggggagagtgt |
| 61 | Divmtqspssltvtagekvtmscksssqsllnsgnqknyltwyqqkpgqppklliywastresgvpdrftgsgsgtdftltissv qaedlav Yycqndysypltfgagtklelkrtvaapsvfifppsdeqlksgtasvvcllnnfypreakvqwkvdnalqsgnsqe svteqdskdstyslsstltlskadyekhkvyacevthqglsspvtksfnrgec |
| 62 | Qiqlvqsgpelkkpgetvkisckasgytftnygmnwvkqapgkglkwmgwintntgeptyaeefkgrfafsletsastayl qinnlkNedtatyfcarlgfgnamdywgqgtsvtvssastkgpsvfplapsskstsggtaalgclvkdyfpepvtvswnsga ltsgvhtfpavlqSsglyslssvvtvpssslgtqtyicnvnhkpsntkvdkkvepkscdkthtcppcpapellggpsvflfppk pkdtlmisrtpevtcvvvdVshedpevkfnwyvdgvevhnaktkpreeqynstyrvvsvltvlhqdwlngkeykckvsn kalpapiektiskakgqprepqvytlPpsrdeltknqvsltclvkgfypsdiavewesngqpennykttppvldsdgsfflysk ltvdksrwqqgnvfscsvmhealhnhytqkslslspgk |
| 63 | Divmtqspssltvtagekvtmscksssqsllnsgnqknyltwyqqkpgqppklliywastresgvpdrftgsgsgtdftltissv qaedlaVyycqndysypftfgsgtkleikrtvaapsvfifppsdeqlksgtasvvcllnnfypreakvqwkvdnalqsgnsqe svteqdskdstyslsstltlskadyekhkvyacevthqglsspvtksfnrgec |
| 64 | Qvqlqqpgaelvrpgasvklsckasgytftsywinwvkqrpgqglewigniypsdsytnynqkfkdkatltvdkssstaym qlsspTsedsavyyctrswrgnsfdywgqgttltvssastkgpsvfplapsskstsggtaalgclvkdyfpepvtvswnsgalt sgvhtfpavlqSsglyslssvvtvpssslgtqtyicnvnhkpsntkvdkkvepkscdkthtcppcpapellggpsvflfppkpk dtlmisrtpevtcvvvdVshedpevkfnwyvdgvevhnaktkpreeqynstyrvvsvltvlhqdwlngkeykckvsnkal papiektiskakgqprepqvytlPpsrdeltknqvsltclvkgfypsdiavewesngqpennykttppvldsdgsfflyskltv dksrwqqgnvfscsvmhealhnhytqkslslspgk |

All documents mentioned in the present application are hereby incorporated by reference in their entireties, as if each is incorporated by reference. In addition, it should be understood that after reading the teachings of the present invention described above, a skilled person in the art can make various changes or modifications of the invention, and these equivalent forms also fall into the scope as defined by the appended claims of the present application.

## Claims

1. A method for treating a CLDN18-positive tumors, comprising administering an antibody that specifically recognize CLDN18 and a chemotherapeutic drug to an individual in need thereof, and the chemotherapeutic drug is:
5-fluorouracil or a prodrug or active metabolite thereof, and oxaliplatin or a prodrug or active metabolite thereof; or 5-fluorouracil or a prodrug or active metabolite thereof, oxaliplatin or a prodrugs or active metabolite thereof and taxanes;
preferably, the chemotherapeutic drug is:
5-fluorouracil or a prodrug thereof, and oxaliplatin or a prodrug thereof; or 5-fluorouracil or a prodrug thereof, oxaliplatin or a prodrug thereof and taxanes;
more preferably, the prodrug of 5-fluorouracil is capecitabine.

2. The method of claim 1, wherein the antibody specifically recognizes CLDN 18.2, instead of CLDN 18.1.

3. The method of claim 1 or 2, wherein the chemotherapeutic drug and the antibody against CLDN18 are administered without a particular order.

4. The method of claim 3, wherein the chemotherapeutic drug is administered, and then the antibody against CLDN18 is administered.

5. The method of claim 4, wherein the chemotherapeutic drug and the antibody against CLDN18 are administered on the same day; or the antibody against CLDN18 is administered at least one day after the chemotherapeutic drug is administered.

6. The method of any one of claims 1-5, wherein the administration dosage of the antibody against CLDN18 is 500-1200 mg/m²/time; preferably, 500-1000 mg/m²/time; more preferably, 500-900 mg/m²/time; and the most preferably, 600-800 mg/m²/time.

7. The method of any one of claims 1-5, wherein the administration dosage of the antibody against CLDN18 is 20-300 mg/kg/time; preferably, 20-100 mg/kg/time; and more preferably, 20-50 mg/kg/time.

8. The method of any one of claims 1-5, wherein the administration dosage of 5-fluorouracil or a prodrug or active metabolite thereof is 300-900 mg/m²/time;
and preferably, 5-fluorouracil is administered at a dosage of 300-800 mg/m²/time; or capecitabine is administered at 600-700 mg/m²/time; and more preferably, 625 mg/m²/time.

9. The method of any one of claims 1-5, wherein the administration dosage of 5-fluorouracil or a prodrug or active metabolite thereof is 50-70 mg/kg/time;
preferably, 5-fluorouracil is administered at a dosage of 50-70 mg/kg/time;
and more preferably, the administration dosage of 5-fluorouracil is 50-60 mg/kg/time.

10. The method of any one of claims 1-5, wherein the administration dosage of oxaliplatin or a prodrug or active metabolite thereof is 50-300 mg/m²/time;
preferably, oxaliplatin is administered at 100-200 mg/m²/time; and more preferably, oxaliplatin is administered at 130 mg/m²/time.

11. The method of any one of claims 1-5, wherein the administration dosage of oxaliplatin or a prodrug or active metabolite thereof is 1-10 mg/kg/time; and preferably, oxaliplatin is administered at 1 to 5 mg/kg/time.

12. The method of any one of claims 1-5, wherein the antibody is a humanized antibody or a chimeric antibody.

13. The method of any one of claims 1-5, wherein the antibody that specifically recognizes CLDN18 comprises:
HCDR1 shown in the amino acid sequence of SEQ ID NO: 16, HCDR2 shown in the amino acid sequence of SEQ ID NO: 17, 40, or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 22, HCDR2 shown in the amino acid sequence of SEQ ID NO: 23, HCDR3 shown in the amino acid sequence of SEQ ID NO: 24, and LCDR1 shown in the amino acid sequence of SEQ ID NO: 25, LCDR2 shown in the amino acid sequence of SEQ ID NO: 26, LCDR3 shown in the amino acid sequence of SEQ ID NO: 27; or
HCDR1 shown in the amino acid sequence of SEQ ID NO: 28, HCDR2 shown in the amino acid sequence of SEQ ID NO: 29, HCDR3 shown in the amino acid sequence of SEQ ID NO: 30, and LCDR1 shown in the amino acid sequence of SEQ ID NO: 31, LCDR2 shown in the amino acid sequence of SEQ ID NO: 32, LCDR3 shown in the amino acid sequence of SEQ ID NO: 33; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48; or
HCDR1 shown in the amino acid sequence of SEQ ID NO: 49, HCDR2 shown in the amino acid sequence of SEQ ID NO: 50, HCDR3 shown in the amino acid sequence of SEQ ID NO: 51, and LCDR1 shown in the amino acid sequence of SEQ ID NO: 52, LCDR2 shown in the amino acid sequence of SEQ ID NO: 53, LCDR3 shown in the amino acid sequence of SEQ ID NO: 54;
preferably, the antibody that specifically recognizes CLDN18 comprises:
HCDR1 shown in the amino acid sequence of SEQ ID NO: 16, HCDR2 shown in the amino acid sequence of SEQ ID NO: 17 or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48.

14. The method of claim 13, wherein the antibody that specifically recognizes CLDN18 comprises:
the heavy chain variable region shown in the amino acid sequence of SEQ ID NO: 2, 9 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 4 and the light chain variable region shown in the amino acid sequence of SEQ ID NO: 3; or
the heavy chain variable region shown in the amino acid sequence of SEQ ID NO: 6 and the light chain variable region shown in the amino acid sequence of SEQ ID NO: 5; or
the heavy chain variable region shown in the amino acid sequence of SEQ ID NO: 55 and the light chain variable region shown in the amino acid sequence of SEQ ID NO: 56; or
the heavy chain variable region shown in the amino acid sequence of SEQ ID NO: 55 and the light chain variable region shown in the amino acid sequence of SEQ ID NO: 56;
preferably, the antibody that specifically recognizes CLDN18 comprises:
the heavy chain variable region shown in the amino acid sequence of SEQ ID NO: 2 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56;
and more preferably, the antibody comprises:
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56.

15. The method of claim 14, wherein the antibody is selected from:
(a) the heavy chain encoded by the nucleotide sequence shown in SEQ ID NO: 59 and the light chain encoded by the nucleotide sequence shown in SEQ ID NO: 60; or,
(b) the heavy chain amino acid sequence shown in SEQ ID NO: 64 and the light chain amino acid sequence shown in SEQ ID NO: 63.

16. The method of claim 1, wherein the taxanes are selected from the group consisting of paclitaxel, albumin-bound paclitaxel and docetaxel; and preferably, albumin-bound paclitaxel.

17. The method of claims 1-16, wherein the CLDN18-positive tumor includes: gastric cancer, pancreatic cancer, esophageal cancer and lung cancer; and preferably, gastric cancer.

18. A combination of kits for treating a CLDN18-positive tumor, wherein the combination of kits includes kit A and kit B, the kit A includes an antibody that specifically recognizes CLDN18, the kit B includes chemotherapeutic drugs, and the chemotherapeutic drugs are: 5-fluorouracil or a prodrug or active metabolite thereof, oxaliplatin or a prodrug or active metabolite thereof; or 5-fluorouracil or a prodrug or active metabolite thereof, oxaliplatin or a prodrug or active metabolite thereof, and taxanes;
Preferably, the chemotherapeutic drugs are:
5-fluorouracil or a prodrug thereof, oxaliplatin or a prodrug thereof; or 5-fluorouracil or a prodrug thereof, oxaliplatin or a prodrug thereof, and taxanes;
And more preferably, the prodrug of 5-fluorouracil is capecitabine.

19. The combination of kits of claim 18, wherein the antibody that specifically recognizes CLDN 18 in the kit A specifically recognizes CLDN 18.2; and preferably, the antibody that specifically recognizes CLDN 18 does not recognizes CLDN 18.1.

20. The combination of kits of claim 18 or 19, wherein the kit A and the kit B are administered without a particular order.

21. The combination of kits of claim 20, wherein the kit B is administered, and then the kit A is administered.

22. The combination of kits of claim 21, wherein the kit A and the kit B are administered on the same day; or the kit A is administered at least one day after the kit B.

23. The combination of kits of any one of claims 18-22, wherein the administration dosage of the kit A is 500-1200 mg/m²/time; preferably, 500-1000 mg/m²/time; more preferably, 500-900 mg/m²/time; and more preferably, 600-800 mg/m²/time.

24. The combination of kits of any one of claims 18-22, wherein the administration dosage of kit A is 20-300 mg/kg/time; preferably, 20-100 mg/kg/time; and more preferably, 20-50 mg/kg/time.

25. The combination of kits of any one of claims 18-22, wherein the administration dosage of 5-fluorouracil or a prodrug or active metabolite thereof is 300-900 mg/m²/time;
Preferably, 5-fluorouracil is administered at a dosage of 300-800 mg/m²/time; or capecitabine is administered at 600-700 mg/m²/time; and preferably, 625 mg/m²/time.

26. The combination of kits of any one of claims 18-22, wherein the administration dosage of 5-fluorouracil or a prodrug or active metabolite thereof is 50-70 mg/kg/time;
preferably, 5-fluorouracil is administered at a dosage of 50-70 mg/kg/time;
and more preferably, 5-fluorouracil is administered at a dosage of 50-60 mg/kg/time.

27. The combination of kits of any one of claims 18-22, wherein the administration dosage of oxaliplatin or a prodrug or active metabolite thereof is 50-300 mg/m²/time;
preferably, oxaliplatin is administered at a dosage of 100-200 mg/m²/time;
and more preferably, oxaliplatin is administered at a dosage of 130 mg/m²/time.

28. The combination of kits of any one of claims 18-22, wherein the administration dosage of oxaliplatin or a prodrug or active metabolite thereof is 1-10 mg/kg/time; and preferably, oxaliplatin is administered at a dosage of 1 to 5 mg/kg/time.

29. The combination of kits of any one of claims 18-22, wherein the antibody that specifically recognizes CLDN18 is a humanized antibody or a chimeric antibody.

30. The combination of kits of any one of claims 18-22, wherein the antibody that specifically recognizes CLDN18 comprises:
HCDR1 shown in the amino acid sequence of SEQ ID NO: 16, HCDR2 shown in the amino acid sequence of SEQ ID NO: 17, 40, or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39; or
HCDR1 shown in the amino acid sequence of SEQ ID NO: 22, HCDR2 shown in the amino acid sequence of SEQ ID NO: 23, HCDR3 shown in the amino acid sequence of SEQ ID NO: 24, and LCDR1 shown in the amino acid sequence of SEQ ID NO: 25, LCDR2 shown in the amino acid sequence of SEQ ID NO: 26, LCDR3 shown in the amino acid sequence of SEQ ID NO: 27; or
HCDR1 shown in the amino acid sequence of SEQ ID NO: 28, HCDR2 shown in the amino acid sequence of SEQ ID NO: 29, HCDR3 shown in the amino acid sequence of SEQ ID NO: 30, and LCDR1 shown in the amino acid sequence of SEQ ID NO: 31, LCDR2 shown in the amino acid sequence of SEQ ID NO: 32, LCDR3 shown in the amino acid sequence of SEQ ID NO: 33; or
HCDR1 shown in the amino acid sequence of SEQ ID NO: 43, HCDR2 shown in the amino acid sequence of SEQ ID NO: 44, HCDR3 shown in the amino acid sequence of SEQ ID NO: 45, and LCDR1 shown in the amino acid sequence of SEQ ID NO: 46, LCDR2 shown in the amino acid sequence of SEQ ID NO: 47, LCDR3 shown in the amino acid sequence of SEQ ID NO: 48; or
HCDR1 shown in the amino acid sequence of SEQ ID NO: 49, HCDR2 shown in the amino acid sequence of SEQ ID NO: 50, HCDR3 shown in the amino acid sequence of SEQ ID NO: 51, and LCDR1 shown in the amino acid sequence of SEQ ID NO: 52, LCDR2 shown in the amino acid sequence of SEQ ID NO: 53, LCDR3 shown in the amino acid sequence of SEQ ID NO: 54;
And preferably, the antibody that specifically recognizes CLDN18 comprises:
HCDR1 shown in the amino acid sequence of SEQ ID NO: 16, HCDR2 shown in the amino acid sequence of SEQ ID NO: 17 or 41, HCDR3 shown in the amino acid sequence of SEQ ID NO: 18, and LCDR1 shown in the amino acid sequence of SEQ ID NO: 19, LCDR2 shown in the amino acid sequence of SEQ ID NO: 20, LCDR3 shown in the amino acid sequence of SEQ ID NO: 21; or
HCDR1 shown in the amino acid sequence of SEQ ID NO: 34, HCDR2 shown in the amino acid sequence of SEQ ID NO: 35 or 42, HCDR3 shown in the amino acid sequence of SEQ ID NO: 36, and LCDR1 shown in the amino acid sequence of SEQ ID NO: 37, LCDR2 shown in the amino acid sequence of SEQ ID NO: 38, LCDR3 shown in the amino acid sequence of SEQ ID NO: 39.

31. The combination of kits of claim 30, wherein the antibody that specifically recognizes CLDN18 comprises:
the heavy chain variable region shown in the amino acid sequence of SEQ ID NO: 2, 9 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 4 and the light chain variable region shown in the amino acid sequence of SEQ ID NO: 3; or
the heavy chain variable region shown in the amino acid sequence of SEQ ID NO: 6 and the light chain variable region shown in the amino acid sequence of SEQ ID NO: 5; or
the heavy chain variable region shown in the amino acid sequence of SEQ ID NO: 55 and the light chain variable region shown in the amino acid sequence of SEQ ID NO: 56; or
the heavy chain variable region shown in the amino acid sequence of SEQ ID NO: 57 and the light chain variable region shown in the amino acid sequence of SEQ ID NO: 58;
preferably, the antibody that specifically recognizes CLDN18 comprises:
the heavy chain variable region shown in the amino acid sequence of SEQ ID NO: 2 or 10 and the light chain variable region shown in the amino acid sequence of SEQ ID NO: 1; or
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 8 and the light chain variable region shown in the amino acid sequence of SEQ ID NO: 7; or
the heavy chain variable region shown in the amino acid sequence of SEQ ID NO: 14 or 15 and the light chain variable region shown in the amino acid sequence of SEQ ID NO: 13; or
the heavy chain variable region shown in the amino acid sequence of SEQ ID NO: 55 and the light chain variable region shown in the amino acid sequence of SEQ ID NO: 56;
and more preferably, the antibody comprises:
the heavy chain variable region shown in the amino acid sequence of SEQ ID NO: 12 and the light chain variable region shown in the amino acid sequence of SEQ ID NO: 11; or
the heavy chain variable region shown in the amino acid sequence of SEQ ID NO: 15 and the light chain variable region shown in the amino acid sequence of SEQ ID NO: 13;
the heavy chain variable region shown in the amino acid sequence of SEQ ID NO: 55 and the light chain variable region shown in the amino acid sequence of SEQ ID NO: 56.

32. The combination of kits of claim 31, wherein the antibody is selected from:
(a) the heavy chain encoded by the nucleotide sequence shown in SEQ ID NO: 59 and the light chain encoded by the nucleotide sequence shown in SEQ ID NO: 60; or
(b) the heavy chain amino acid sequence shown in SEQ ID NO: 64 and the light chain amino acid sequence shown in SEQ ID NO: 63.

33. The combination of kits of claim 18, wherein the taxanes in the kit B are selected from the group consisting of paclitaxel, albumin-bound paclitaxel and docetaxel; and preferably, albumin-bound paclitaxel.

34. The combination of kits of any one of claims 18-33, wherein the CLDN18-positive tumor includes: gastric cancer, pancreatic cancer, esophageal cancer and lung cancer.

35. A pharmaceutical composition for treating a CLDN18-positive tumor, wherein the pharmaceutical composition comprises an antibody that specifically recognizes CLDN18 and chemotherapeutic drugs, and the chemotherapeutic drugs are 5-fluorouracil or a prodrug or active metabolite thereof, oxaliplatin or a prodrug or active metabolite thereof; or, 5-fluorouracil or a prodrug or active metabolite thereof, oxaliplatin or a prodrug or active metabolite thereof, and taxanes, as described in any one of preceding claims; and the antibody that specifically recognizes CLDN18 is the antibody as described in any one of preceding claims.

36. Use of the combination of kits of any one of claims 18-34 or the pharmaceutical composition of claim 35 in the preparation of a medicament for treating a CLDN18-positive tumor.

37. The combination of kits of any one of claims 18-34 or the pharmaceutical composition of claim 35 for use in treating a CLDN18-positive tumor.


Sequence listing

<110>  CAFA THERAPEUTICS LIMITED

<120>  COMBINATION THERAPY OF CLDN18 ANTIBODY AND CHEMOTHERAPY DRUGS

<130>  X7-B-63119 EP

<140>  PCT/CN2019/109214
<141>  2019-09-29

<150>  CN201811598638
<151>  2018-09-30

<150>  CN201811246053
<151>  2018-10-19

<160>  64

<170>  PatentIn version 3.5

<210>  1
<211>  114
<212>  PRT
<213>  Artificial sequence

<220>
<223>  Synthesized polypeptide

<400>  1

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210>  2
<211>  118
<212>  PRT
<213>  Artificial sequence

<220>
<223>  Synthesized polypeptide

<400>  2

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115


<210>  3
<211>  107
<212>  PRT
<213>  Artificial sequence

<220>
<223>  Synthesized polypeptide

<400>  3

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Ser Tyr Met
            20                  25                  30

His Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Pro Tyr Thr
                85                  90                  95

Phe Gly Gly Gly Thr Lys Leu Glu Ile Lys Arg
            100                 105

<210>  4
<211>  120
<212>  PRT
<213>  Artificial sequence

<220>
<223>  Synthesized polypeptide

<400>  4

Gln Val Gln Leu Gln Gln Ser Gly Pro Glu Leu Val Lys Pro Gly Ala
1               5                   10                  15

Leu Val Lys Ile Ser Cys Lys Ala Ser Gly Tyr Thr Phe Thr Ser Tyr
            20                  25                  30

Asp Ile Asn Trp Val Lys Gln Arg Pro Gly Gln Gly Leu Glu Trp Ile
        35                  40                  45

Gly Trp Ile Tyr Pro Gly Asp Gly Ser Thr Lys Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asn Ser Ala Val Tyr Phe Cys
                85                  90                  95

Ala Arg Gly Gly Tyr Arg Tyr Asp Glu Ala Met Asp Tyr Trp Gly Gln
            100                 105                 110

Gly Thr Thr Val Thr Val Ser Ser
        115                 120

<210>  5
<211>  114
<212>  PRT
<213>  Artificial sequence

<220>
<223>  Synthesized polypeptide

<400>  5

Asp Ile Val Met Thr Gln Ser Pro Ser Ser Leu Ser Val Ser Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Ala Trp Tyr Gln Gln Lys Pro Gly Gln
        35                  40                  45

Pro Pro Lys Leu Leu Ile Tyr Gly Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp His Ser Tyr Pro Leu Thr Phe Gly Ala Gly Thr Lys Leu Glu Leu
            100                 105                 110

Lys Arg

<210>  6
<211>  118
<212>  PRT
<213>  Artificial sequence

<220>
<223>  Synthesized polypeptide

<400>  6

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Phe Ser Tyr Gly Asn Ser Phe Ala Tyr Trp Gly Gln Gly Thr
            100                 105                 110

Thr Val Thr Val Ser Ser
        115

<210>  7
<211>  114
<212>  PRT
<213>  Artificial sequence

<220>
<223>  Synthesized polypeptide

<400>  7

Asp Ile Val Met Thr Gln Ser Pro Ser Ser Leu Thr Val Thr Pro Gly
1               5                   10                  15

Glu Lys Val Thr Met Thr Cys Lys Ser Ser Gln Ser Leu Phe Asn Ser
            20                  25                  30

Gly Asn Gln Lys Asn Tyr Leu Thr Trp Tyr Gln Gln Arg Pro Gly Gln
        35                  40                  45

Pro Pro Lys Met Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Phe Tyr Cys Gln Asn
                85                  90                  95

Ala Tyr Ser Phe Pro Tyr Thr Phe Gly Gly Gly Thr Lys Leu Glu Ile
            100                 105                 110

Lys Arg

<210>  8
<211>  118
<212>  PRT
<213>  Artificial sequence

<220>
<223>  Synthesized polypeptide

<400>  8

Asp Val Gln Leu Gln Glu Ser Gly Pro Asp Leu Val Lys Pro Ser Gln
1               5                   10                  15

Ser Leu Ser Leu Thr Cys Thr Val Thr Gly Tyr Ser Ile Thr Ser Gly
            20                  25                  30

Tyr Asn Trp His Trp Ile Arg Gln Phe Pro Gly Asn Lys Met Glu Trp
        35                  40                  45

Met Gly Tyr Ile His Tyr Thr Gly Ser Thr Asn Tyr Asn Pro Ser Leu
    50                  55                  60

Arg Ser Arg Ile Ser Ile Thr Arg Asp Thr Ser Lys Asn Gln Phe Phe
65                  70                  75                  80

Leu Gln Leu Asn Ser Val Thr Thr Asp Asp Thr Ala Thr Tyr Tyr Cys
                85                  90                  95

Thr Arg Ile Tyr Asn Gly Asn Ser Phe Pro Tyr Trp Gly Gln Gly Thr
            100                 105                 110

Ser Val Thr Val Ser Ser
        115


<210>  9
<211>  118
<212>  PRT
<213>  Artificial sequence

<220>
<223>  Synthesized polypeptide

<400>  9

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asp Pro Ser Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  10
<211>  118
<212>  PRT
<213>  Artificial sequence

<220>
<223>  Synthesized polypeptide

<400>  10

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ala Ser Gly Tyr Thr Asn Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Tyr Tyr Gly Asn Ser Phe Ala Tyr Trp Gly Gln Gly Thr
            100                 105                 110

Thr Val Thr Val Ser Ser
        115

<210>  11
<211>  114
<212>  PRT
<213>  Artificial sequence

<220>
<223>  Synthesized polypeptide

<400>  11

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Asp Tyr Ser Tyr Pro Leu Thr Phe Gly Gly Gly Thr Lys Val Glu Ile
            100                 105                 110

Lys Arg



<210>  12
<211>  118
<212>  PRT
<213>  Artificial sequence

<220>
<223>  Synthesized polypeptide

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Thr Met His Trp Val Arg Gln Ala Pro Gly Gln Gly Leu Glu Trp Met
        35                  40                  45

Gly Tyr Ile Asn Pro Ala Ser Gly Tyr Thr Asn Tyr Asn Gln Lys Phe
    50                  55                  60

Lys Asp Arg Val Thr Met Thr Arg Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ile Tyr Tyr Gly Asn Ser Phe Ala Tyr Trp Gly Gln Gly Thr
            100                 105                 110

Leu Val Thr Val Ser Ser
        115

<210>  13
<211>  114
<212>  PRT
<213>  Artificial sequence

<220>
<223>  Synthesized polypeptide

<400>  13

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Phe Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Ala Tyr Ser Phe Pro Tyr Thr Phe Gly Gly Gly Thr Lys Leu Glu Ile
            100                 105                 110

Lys Arg

<210>  14
<211>  118
<212>  PRT
<213>  Artificial sequence

<220>
<223>  Synthesized polypeptide

<400>  14

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser
        115


<210>  15
<211>  118
<212>  PRT
<213>  Artificial sequence

<220>
<223>  Synthesized polypeptide

<400>  15

Gln Val Gln Leu Gln Glu Ser Gly Pro Gly Leu Ile Lys Pro Ser Gln
1               5                   10                  15

Thr Leu Ser Leu Thr Cys Thr Val Ser Gly Gly Ser Ile Ser Ser Gly
            20                  25                  30

Tyr Asn Trp His Trp Ile Arg Gln Pro Pro Gly Lys Gly Leu Glu Trp
        35                  40                  45

Ile Gly Tyr Ile His Tyr Thr Gly Ser Thr Asn Tyr Asn Pro Ala Leu
    50                  55                  60

Arg Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Ile Tyr Tyr Cys
                85                  90                  95

Ala Arg Ile Tyr Asn Gly Asn Ser Phe Pro Tyr Trp Gly Gln Gly Thr
            100                 105                 110

Thr Val Thr Val Ser Ser
        115

<210>  16
<211>  5
<212>  PRT
<213>  Artificial sequence

<220>
<223>  Synthesized polypeptide

<400>  16

Ser Tyr Thr Met His
1               5

<210>  17
<211>  17
<212>  PRT
<213>  Artificial sequence

<220>
<223>  Synthesized polypeptide

<400>  17

Tyr Ile Asn Pro Ser Ser Gly Tyr Thr Asn Tyr Asn Gln Lys Phe Lys
1               5                   10                  15

Asp

<210>  18
<211>  9
<212>  PRT
<213>  Artificial sequence

<220>
<223>  Synthesized polypeptide

<400>  18

Ile Tyr Tyr Gly Asn Ser Phe Ala Tyr
1               5

<210>  19
<211>  17
<212>  PRT
<213>  Artificial sequence

<220>
<223>  Synthesized polypeptide

<400>  19

Lys Ser Ser Gln Ser Leu Leu Asn Ser Gly Asn Gln Lys Asn Tyr Leu
1               5                   10                  15

Thr

<210>  20
<211>  7
<212>  PRT
<213>  Artificial sequence

<220>
<223>  Synthesized polypeptide

<400>  20

Trp Ala Ser Thr Arg Glu Ser
1               5

<210>  21
<211>  9
<212>  PRT
<213>  Artificial sequence

<220>
<223>  Synthesized polypeptide

<400>  21

Gln Asn Asp Tyr Ser Tyr Pro Leu Thr
1               5

<210>  22
<211>  5
<212>  PRT
<213>  Artificial sequence

<220>
<223>  Synthesized polypeptide

<400>  22

Ser Tyr Asp Ile Asn
1               5

<210>  23
<211>  17
<212>  PRT

<213>  Artificial sequence

<220>
<223>  Synthesized polypeptide

<400>  23

Trp Ile Tyr Pro Gly Asp Gly Ser Thr Lys Tyr Asn Glu Lys Phe Lys
1               5                   10                  15

Gly

<210>  24
<211>  11
<212>  PRT
<213>  Artificial sequence

<220>
<223>  Synthesized polypeptide

<400>  24

Gly Gly Tyr Arg Tyr Asp Glu Ala Met Asp Tyr
1               5                   10

<210>  25
<211>  10
<212>  PRT
<213>  Artificial sequence

<220>
<223>  Synthesized polypeptide

<400>  25

Ser Ala Ser Ser Ser Ile Ser Tyr Met His
1               5                   10

<210>  26
<211>  7
<212>  PRT
<213>  Artificial sequence

<220>
<223>  Synthesized polypeptide

<400>  26

Asp Thr Ser Lys Leu Ala Ser
1               5

<210>  27
<211>  9
<212>  PRT
<213>  Artificial sequence

<220>

<223>  Synthesized polypeptide

<400>  27

His Gln Arg Ser Ser Tyr Pro Tyr Thr
1               5


<210>  28
<211>  5
<212>  PRT
<213>  Artificial sequence

<220>
<223>  Synthesized polypeptide

<400>  28

Asn Tyr Gly Met Asn
1               5


<210>  29
<211>  17
<212>  PRT
<213>  Artificial sequence

<220>
<223>  Synthesized polypeptide

<400>  29

Trp Ile Asn Thr Asn Thr Gly Glu Pro Thr Tyr Ala Glu Glu Phe Lys
1               5                   10                  15


Gly



<210>  30
<211>  9
<212>  PRT
<213>  Artificial sequence

<220>
<223>  Synthesized polypeptide

<400>  30

Phe Ser Tyr Gly Asn Ser Phe Ala Tyr
1               5


<210>  31
<211>  17
<212>  PRT
<213>  Artificial sequence

<220>
<223>  Synthesized polypeptide

<400>  31

Lys Ser Ser Gln Ser Leu Leu Asn Ser Gly Asn Gln Lys Asn Tyr Leu
1               5                   10                  15

Ala

<210>  32
<211>  7
<212>  PRT
<213>  Artificial sequence

<220>
<223>  Synthesized polypeptide

<400>  32

Gly Ala Ser Thr Arg Glu Ser
1               5

<210>  33
<211>  9
<212>  PRT
<213>  Artificial sequence

<220>
<223>  Synthesized polypeptide

<400>  33

Gln Asn Asp His Ser Tyr Pro Leu Thr
1               5

<210>  34
<211>  6
<212>  PRT
<213>  Artificial sequence

<220>
<223>  Synthesized polypeptide

<400>  34

Ser Gly Tyr Asn Trp His
1               5

<210>  35
<211>  16
<212>  PRT
<213>  Artificial sequence

<220>
<223>  Synthesized polypeptide

<400>  35

Tyr Ile His Tyr Thr Gly Ser Thr Asn Tyr Asn Pro Ser Leu Arg Ser
1               5                   10                  15

<210>  36
<211>  9
<212>  PRT
<213>  Artificial sequence

<220>
<223>  Synthesized polypeptide

<400>  36

Ile Tyr Asn Gly Asn Ser Phe Pro Tyr
1               5


<210>  37
<211>  17
<212>  PRT
<213>  Artificial sequence

<220>
<223>  Synthesized polypeptide

<400>  37

Lys Ser Ser Gln Ser Leu Phe Asn Ser Gly Asn Gln Lys Asn Tyr Leu
1               5                   10                  15


Thr




<210>  38
<211>  7
<212>  PRT
<213>  Artificial sequence

<220>
<223>  Synthesized polypeptide

<400>  38

Trp Ala Ser Thr Arg Glu Ser
1               5


<210>  39
<211>  9
<212>  PRT
<213>  Artificial sequence

<220>
<223>  Synthesized polypeptide

<400>  39

Gln Asn Ala Tyr Ser Phe Pro Tyr Thr
1               5


<210>  40

<211>  17
<212>  PRT
<213>  Artificial sequence

<220>
<223>  Synthesized polypeptide

<400>  40

Tyr Ile Asp Pro Ser Ser Gly Tyr Thr Asn Tyr Asn Gln Lys Phe Lys
1               5                   10                  15

Asp

<210>  41
<211>  17
<212>  PRT
<213>  Artificial sequence

<220>
<223>  Synthesized polypeptide

<400>  41

Tyr Ile Asn Pro Ala Ser Gly Tyr Thr Asn Tyr Asn Gln Lys Phe Lys
1               5                   10                  15

Asp

<210>  42
<211>  16
<212>  PRT
<213>  Artificial sequence

<220>
<223>  Synthesized polypeptide

<400>  42

Tyr Ile His Tyr Thr Gly Ser Thr Asn Tyr Asn Pro Ala Leu Arg Ser
1               5                   10                  15

<210>  43
<211>  5
<212>  PRT
<213>  Artificial sequence

<220>
<223>  Synthesized polypeptide

<400>  43

Ser Tyr Trp Ile Asn
1               5

<210>  44
<211>  17
<212>  PRT
<213>  Artificial sequence

<220>
<223>  Synthesized polypeptide

<400>  44

Asn Ile Tyr Pro Ser Asp Ser Tyr Thr Asn Tyr Asn Gln Lys Phe Lys
1               5                   10                  15

Asp

<210>  45
<211>  9
<212>  PRT
<213>  Artificial sequence

<220>
<223>  Synthesized polypeptide

<400>  45

Ser Trp Arg Gly Asn Ser Phe Asp Tyr
1               5

<210>  46
<211>  17
<212>  PRT
<213>  Artificial sequence

<220>
<223>  Synthesized polypeptide

<400>  46

Lys Ser Ser Gln Ser Leu Leu Asn Ser Gly Asn Gln Lys Asn Tyr Leu
1               5                   10                  15

Thr

<210>  47
<211>  7
<212>  PRT
<213>  Artificial sequence

<220>
<223>  Synthesized polypeptide

<400>  47

Trp Ala Ser Thr Arg Glu Ser
1               5

<210>  48
<211>  9
<212>  PRT
<213>  Artificial sequence

<220>
<223>  Synthesized polypeptide

<400>  48

Gln Asn Asp Tyr Ser Tyr Pro Phe Thr
1               5


<210>  49
<211>  5
<212>  PRT
<213>  Artificial sequence

<220>
<223>  Synthesized polypeptide

<400>  49

Asn Tyr Gly Met Asn
1               5


<210>  50
<211>  17
<212>  PRT
<213>  Artificial sequence

<220>
<223>  Synthesized polypeptide

<400>  50

Trp Ile Asn Thr Asn Thr Gly Glu Pro Thr Tyr Ala Glu Glu Phe Lys
1               5                   10                  15


Gly


<210>  51
<211>  9
<212>  PRT
<213>  Artificial sequence

<220>
<223>  Synthesized polypeptide

<400>  51

Leu Gly Phe Gly Asn Ala Met Asp Tyr
1               5


<210>  52
<211>  17

<212>  PRT
<213>  Artificial sequence

<220>
<223>  Synthesized polypeptide

<400>  52

Lys Ser Ser Gln Ser Leu Leu Asn Ser Gly Asn Gln Lys Asn Tyr Leu
1               5                   10                  15

Thr

<210>  53
<211>  7
<212>  PRT
<213>  Artificial sequence

<220>
<223>  Synthesized polypeptide

<400>  53

Trp Ala Ser Thr Arg Glu Ser
1               5

<210>  54
<211>  9
<212>  PRT
<213>  Artificial sequence

<220>
<223>  Synthesized polypeptide

<400>  54

Gln Asn Asp Tyr Ser Tyr Pro Leu Thr
1               5

<210>  55
<211>  118
<212>  PRT
<213>  Artificial sequence

<220>
<223>  Synthesized polypeptide

<400>  55

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile

35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser
        115

<210>  56
<211>  114
<212>  PRT
<213>  Artificial sequence

<220>
<223>  Synthesized polypeptide

<400>  56

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Phe Thr Phe Gly Ser Gly Thr Lys Leu Glu Ile
            100                 105                 110

Lys Arg

<210>  57
<211>  114
<212>  PRT
<213>  Artificial sequence

<220>
<223>  Synthesized polypeptide

<400>  57

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg

<210>  58
<211>  118
<212>  PRT
<213>  Artificial sequence

<220>
<223>  Synthesized polypeptide

<400>  58

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met

35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Leu Gly Phe Gly Asn Ala Met Asp Tyr Trp Gly Gln Gly Thr
            100                 105                 110

Ser Val Thr Val Ser Ser
        115

<210>  59
<211>  1344
<212>  DNA
<213>  Artificial sequence

<220>
<223>  Synthesized polynucleotide

<400>  59
caggtgcagc tgcaggagag cggccccggc ctgatcaagc ccagccagac cctgagcctg      60

acctgcaccg tgagcggcgg cagcatcagc agcggctaca actggcactg gatccggcag     120

ccccccggca agggcctgga gtggatcggc tacatccact acaccggcag caccaactac     180

aaccccgccc tgcggagccg ggtgaccatc agcgtggaca ccagcaagaa ccagttcagc     240

ctgaagctga gcagcgtgac cgccgccgac accgccatct actactgcgc ccggatctac     300

aacggcaaca gcttccccta ctggggccag ggcaccaccg tgaccgtgag cagcgctagc     360

accaaaggcc catcggtctt ccccctggca ccctcctcca agagcacctc tgggggcaca     420

gcggccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gaccgtgccc tccagcagct tgggcaccca gacctacatc     600

tgcaacgtga atcacaagcc cagcaacacc aaggtggaca agaaagttga gcccaaatct     660

tgtgacaaaa ctcacacatg cccaccgtgc ccagcacctg aactcctggg gggaccgtca     720

gtcttcctct tccccccaaa acccaaggac accctcatga tctcccggac ccctgaggtc     780

acatgcgtgg tggtggacgt gagccacgaa gaccctgagg tcaagttcaa ctggtacgtg     840

gacggcgtgg aggtgcataa tgccaagaca aagccgcggg aggagcagta caacagcacg     900

taccgtgtgg tcagcgtcct caccgtcctg caccaggact ggctgaatgg caaggagtac     960

aagtgcaagg tctccaacaa agccctccca gcccccatcg agaaaaccat ctccaaagcc 1020

aaagggcagc cccgagaacc acaggtgtac accctgcccc catcccggga tgagctgacc 1080

aagaaccagg tcagcctgac ctgcctggtc aaaggcttct atcccagcga catcgccgtg 1140

gagtgggaga gcaatgggca gccggagaac aactacaaga ccacgcctcc cgtgctggac 1200

tccgacggct ccttcttcct ctatagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaa 1344

<210> 60
<211> 660
<212> DNA
<213> Artificial sequence

<220>
<223> Synthesized polynucleotide

<400> 60
gacatcgtga tgacccagag ccccgacagc ctggccgtga gcctgggcga gcgggccacc 60

atcaactgca agagcagcca gagcctgttc aacagcggca accagaagaa ctacctgacc 120

tggtaccagc agaagcccgg ccagcccccc aagctgctga tctactgggc cagcacccgg 180

gagagcggcg tgcccgaccg gttcagcggc agcggcagcg gcaccgactt caccctgacc 240

atcagcagcc tgcaggccga ggacgtggcc gtgtactact gccagaacgc ctacagcttc 300

ccctacacct tcggcggcgg caccaagctg gagatcaagc ggacggtggc tgcaccatct 360

gtcttcatct tcccgccatc tgatgagcag ttgaaatctg gaactgcctc tgttgtgtgc 420

ctgctgaata acttctatcc cagagaggcc aaagtacagt ggaaggtgga taacgccctc 480

caatcgggta actcccagga gagtgtcaca gagcaggaca gcaaggacag cacctacagc 540

ctcagcagca ccctgacgct gagcaaagca gactacgaga aacacaaagt ctacgcctgc 600

gaagtcaccc atcagggcct gagctcgccc gtcacaaaga gcttcaacag gggagagtgt 660

<210> 61
<211> 220
<212> PRT
<213> Artificial sequence

<220>
<223> Synthesized polypeptide

<400> 61

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser

20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Leu Thr Phe Gly Ala Gly Thr Lys Leu Glu Leu
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  62
<211>  448
<212>  PRT
<213>  Artificial sequence

<220>
<223>  Synthesized polypeptide

<400>  62

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Phe Thr Asn Tyr
            20                  25                  30

Gly Met Asn Trp Val Lys Gln Ala Pro Gly Lys Gly Leu Lys Trp Met
        35                  40                  45

Gly Trp Ile Asn Thr Asn Thr Gly Glu Pro Thr Tyr Ala Glu Glu Phe
    50                  55                  60

Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95

Ala Arg Leu Gly Phe Gly Asn Ala Met Asp Tyr Trp Gly Gln Gly Thr
            100                 105                 110

Ser Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro

260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  63
<211>  220
<212>  PRT
<213>  Artificial sequence

<220>
<223>  Synthesized polypeptide

<400>  63

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Phe Thr Phe Gly Ser Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  64
<211>  448
<212>  PRT
<213>  Artificial sequence

<220>
<223>  Synthesized polypeptide

<400>  64

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile
        35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445